# EUROPEAN PATENT SPECIFICATION

(11) **EP 2 966 164 B1**
(45) Date of publication and mention of the grant of the patent: **19.04.2017**
(21) Application number: 15002155.8
(22) Date of filing: 23.02.2012
(51) Int. Cl.: C12N 1/20

(54) **ISOLATED MICROORGANISM STRAIN LACTOBACILLUS GASSERI MCC2 DSM23882 AND ITS USE**
ISOLIERTER MIKROORGANISMUSSTAMM LACTOBACILLUS GASSERI MCC2 DSM23882 UND DESSEN
SOUCHE DE MICRO-ORGANISME ISOLÉ DE LACTOBACILLUS GASSERI MCC2 DSM23882 ET SON UTILISATION

(30) Priority: 25.02.2011 EE 201100012
(43) Date of publication of application: 13.01.2016
(62) Divisional of application: 12709275.7
(73) Proprietor: Oü Tervisliku Piima Biotehnoloogiate Arenduskeskus, 51014 Tartu (EE)
(72) Inventor: Tammsaar, Ene, 79702 Rapla maakond (EE); Songisepp, Epp, 50107 Tartu (EE); Rätsep, Merle, 50109 Tartu (EE); Ehrlich, Kersti, 50103 Tartu (EE); Punab, Margus, 51006 Tartu (EE); Vasar, Maire, 51005 Tartu (EE); Mikelsaar, Marika, 51005 Tartu (EE); Bobrovski, Lauri, 73021 Järvamaa (EE); Zilmer, Kersti, 50406 Tartu (EE); Veskioja, Andre, 63221 Põlva maakond (EE); Zilmer, Mihkel, 50406 Tartu (EE)
(74) Representative: Kahu, Sirje

(56) References cited:
- WO-A1-2005/058335
- WO-A2-2006/041930

## Description

### TECHNICAL FIELD

The invention belongs to the field of biotechnology and will be used in food industry. More precisely the invention deals with the microorganism strain *L. gasseri* MCC2 and its use in the reduction of milk allergy and irritation symptoms of lower urinary tract accompanying benign prostate hyperplasia and oxidative stress and inflammation associated with these.

### BACKGROUND ART

Lactobacilli have for a long time been widely used to make healthy food products, for example as starter cultures. The most common way is the use of lactobacilli in functional food. Functional food is a food product that in addition to ordinary dietary values has additional natural components that beneficially influence some functions of the organism or reduce the risk of diseases.

Methods which include purposefully bioselected live lactobacilli that in the following technological stage are killed with pasteurization have been less used in preparing food products. This means that the end product does not contain live lactic acid bacteria, but with pretreatment, suitable technological scheme and necessary additives the created product has purposeful bioeffect.

### Milk allergy and relieving it

Infections, inflammation-, allergy- and oxidative stress-related problems, for example food allergies, urinary tract symptoms (e.g. micturition disorders associated with prostate ailments) are becoming economically more burdensome for the society. For example up to 4% of population is suffering from food allergies and in the USA 100-200 deaths are directly caused by food allergy. Food allergies (including allergic reaction to cow's milk) are an especially serious problem for infants. The use of milk products in infants is essential to guarantee their normal growth and development. The solving of the problem of food allergenicity is very complicated as it is not possible to create products that are absolutely allergic reaction-free and at the same time with very high good bioquality. The last discussions in UN, FAO and WHO have declared - *allergen-free: time to clarity; statement: there is no actual legislation to declare free from all allergens.* Thus: any technological ideas/solutions which result in more hypoallergenic products that would reduce the number of infants with allergic response by at least some percent would be highly rated. Every percent of the reduction of allergic responses is very important in conclusion/longer perspective, as any success in the field of allergy to reduce the allergenicity of food products has significant socio-economic output (including improvement of life quality in children and adults and reduction of treatment costs).

The allergenicity of milk products is determined by proteins, but it is difficult to bring out one so-called main allergen (Encyclopedia of Food Sciences and Nutrition (EFSN) AP, 2003, Wal, J. M. (2004) Bovine milk allergenicity. Ann.Allergy Asthma Immunol., 93, S2-11). Nevertheless both casein and beta-lactoglobulin cause allergic reactions in equal proportion. Cow's milk allergy is mainly associated with α-casein and β-lactoglobulin (Host A. (2002). Frequency of cow's milk allergy in childhood. Ann Allergy Immunol 89(Suppl 1) :33-7; EFSN, 2003. Many children with milk allergy have a reaction to alpha-casein (Hill D.J, Firer M.A, Shelton M.J et al. (1986) Manifestation of milk allergy in infancy: clinical and immunologic findings. J Pediatr 109: 270-6; EFSN, 2003, Vandenplas Y, Brueton M, Dupont C, Hill D, Isolauri E, Koletzko S, Oranje AP, Staiano A. (2007) Guidelines for the diagnosis and management of cow's milk protein allergy in infants. Arch Dis Child. 92:902-908).

Casein consists of the following subunits: α-S1 (main allergen), α-S2, β- and κ-casein, whereat α-S1 and β-casein are predominant. Enzymatic hydrolysis of milk proteins reduces their allergenicity. Large peptides are formed during partial hydrolysis of milk proteins, more complete hydrolysis results in a mixture of large and small peptides and amino acids. Even a thorough pepsin-trypsin hydrolysis does not give an allergy-free result as allergic reaction may be caused by negligible amounts of immunoreactive components of native protein. Thus only a thorough hydrolysis-based approach has created some possibilities, but does not offer a final solution, even in case of hydrolyzates of whey proteins (Businco L, Cantani A, Longhi MA, Giampietro PG. (1989) Anaphylactic reactions to a cow's milk whey protein hydrolysate (Alfa-Ré, Nestle) in infants with cow's milk allergy. Ann Allergy. 62(4):333-5.; Sampson HA, James MJ, Bernhisel-Broadbent J. (1992) Safety of an amino-derived infant formula in children allergic to cow milk. Pediatrics. 90,463-465). Thus new approaches to reduce cow's milk allergy are needed, wherefore directed hydrolysis of allergy-causing milk proteins with the strain(s) of microorganism(s) giving additional values to the products, is a promising trend for significant reduction of allergy problems.

The hydrolysis of milk proteins with the help of lactic acid bacteria and additives has been described previously. In the patent EE03424 (patent application WO9700078, Valio Oy) the use of *L. rhamnosus* ATCC 53103 (LGG) strain is described for the production of protein hydrolysate-preparation, where the proteins are hydrolyzed with pepsin and trypsin. The use of LGG for the reduction of allergy risk is considered in several inventions of Nestec SA. In patent EP1296694 the use of LGG for reduction of atopic diseases risk in children is described. In patent application WO2008116907 the use of bifidobacteria and LGG, *L. rhamnosus* CGMCC 1.3724, *L. reuteri* ATCC 55730 and *L. paracasei* CNCM I-2116 for the reduction of allergy risk in infants born with Caesarean section is described, including with partially (2-20%) hydrolyzed proteins. In patent application WO03099037 by Nestec SA a proteolytic system of lactic acid bacteria and a food product containing *Bifidobacterium lactis* ATCC 27536 is described for use among other things to reduce food allergy while the bacteria may be inactivated or dead. In patent EE04724 (patent application WO9929833, Arla Foods) the use of *L*. *paracasei subsp. paracasei* is described, including for alleviating atopic problems in children. In patent EP1175156 (Bioferme OY) a pasteurized cereal drink containing microorganisms is described. In the patent claim CN101427782 (Min Zhao) bifidobacterium-containing maize drink is described, which is produced with the help of fermentation and where the bacteria are inactivated in the fermented product. In a whey protein concentrate (WPC) the maximum achieved hydrolysis of β-lactoglobulin has been 21% (M.Pescuma et al (2007), Hydrolysis of whey proteins by L. acidophilus, Streptococcus thermophilus and L. delbrueckii ssp. bulgaricus grown in a chemically defined medium, Journal of Appl Microbiol, 103,5,1738-1746). Milk protein hydrolysis (2-32%) by two microorganisms with the aim to receive a product with good gustatory properties, is described in the European patent EP1383394 (New Zealand Dairy Board), where one proposed microorganism is *Micrococcus, Micrococcus, Entercoccus, Staphylococcus, Brevibacterium, Anthrobacter* or *Corynebacterium,* preferably *Macrococcus caseolyticus,* and the other microorganism lactic acid bacterium *Lactococcus, Lactobacillus, Pediococcus* or *Leuconostoc,* and the fermentation is stopped by removal or killing of the microorganisms. In Japanese patent application JP7203844 (Morinaga Milk Industry Co) partial hydrolysis of milk whey (30%) with the help of an enzyme derived from microorganism *Bacillus subtilis,* trypsin and papain is described.

### Prostate complaints and relieving them

Prostate may cause several complaints: urinary obstruction and irritation and pain or discomfort in the region of minor pelvis. Prostate complaints, especially those associated with urination, are very common and reduce the quality of life of many men. It is estimated that prostate hyperplasia (Benign Prostatic Hyperplasia - BPH) occurs in about half of 50-year old men and in about 70% of men over 70. The lower urinary tract symptoms (LUTS) accompanying benign prostatic hyperplasia include irritating and obstructive (voiding) symptoms which are assessed using the International Prostate Symptom Score (IPSS). Every non-pharmaceutical possibility to relieve urinary difficulties of men would be valued and it would be the best if a novel food product would have such effect.

In several patent applications the use of lactic acid bacteria has been described in therapeutic compositions for the treatment and alleviation of urogenital infections, including prostate infection, whereas the compositions contain one or several species of lactic acid bacteria (US2008274162, Nessa Jeffrey Bryan et al., 2007).
The combination of lactic acid bacteria, where one component contains *L. crispatus, L. salivarius* and *L. casei* and the other component contains *L. brevis, L. gasseri* and *L*. *fermentum,* may be used as a dietary supplement or pharmaceutical composition for the treatment and prophylaxis of infections and inflammations, including urethritis (EE04620, *Actial Farmaceutica Lda,* 2000). Lactic acid bacterium *L. coprophilus* PL9001 (KCCM-10245) has been used to prevent and treat urogenital infections (KR20040067161, PL BIO Co Ltd., 2003). Pharmaceutical compositions used in urology for local treatment may contain lactic acid bacteria *L. casei, L. gasseri* as an active substance (EP353581, Silvana Tosi et al., 1993).

This shows that milk allergy and prostate complaints alleviation, including with the help of partial hydrolysis and lactic acid bacteria, has been studied for a long time.

Irrespective of this there are not sufficient amount of food products and dietary supplements that reduce milk allergy and prostate complaints available on the market. Thus there is a need for production and use of reliable, test-proven food products and dietary supplements with healthy properties.

### DISCLOSURE OF THE INVENTION

This invention relates to new isolated antioxidant microorganism strain *L. gasseri MCC2* DSM 23882, composition containing this strain, the use of the mentioned microorganism as an antioxidant proteolytic ingredient for production of food product and dietary supplement, the use of microorganism for production of food product and dietary supplement reducing milk allergy and lower urinary tract irritation symptoms accompanying benign prostatic enlargement and oxidative stress and inflammation associated with these, and method for producing food product and dietary supplement reducing abovementioned complaints.

Lactic acid bacteria partly hydrolyzing milk proteins, that is the object of this invention, were found by gradual bioselection. The aim was to find strains with multivalent biopotential, the multivalency of which would allow the synergistic use of the beneficial bioeffects (more hypoallergenic, less allergic responses, inflammation and oxidative stress reducing effect) to obtain the food products with desired properties. The approach ideology of the invention is not the achievement of complete hydrolysis of proteins. Complete hydrolysis has been attempted, but this creates new problems including a very severe organoleptic unpleasentness of the product. The use of multibiopotent strains with required technical procedures and the use of appropriate healthy additional raw products were chosen as the ideology of the invention. *L. gasseri* MCC2 is multibiopotent and with synergistic effect - it is able to purposefully (partially) hydrolyze milk proteins (caseins), has significant oxidation resistance, produces conjugated linoleic acid, NO and other components. Thus the invention makes it possible with the help of a biotechnologic integral solution to create food products that would have a potential to reduce milk allergy and urinary tract complaints.

### Description of L. gasseri MCC2

The object of the invention, *L. gasseri* MCC2 DSM 23882 was isolated from the faeces of a healthy child during an Estonian-Swedish comparative study of children's microflora. Strain strain *L. gasseri* MCC2 was isolated by plating serial dilutions of faeces of a 1-year old child (10⁻²-10⁻⁷ in phosphate buffer with 0.04% thioglycolic acid, pH 7.2). The dilutions were plated on a freshly prepared MRS agar culture media (Oxoid, UK) and cultivated at 37°C in microaerobic environment. The strains that were the objects of the invention were isolated on the basis of colony and cell morphology characteristic to *Lactobacillus ssp.* This was followed by provisional and thereafter more precise identification described below.

*L. gasseri* MCC2 was deposited in culture collection Deutsche Sammlung von Mikroorganismen und Zellkulturen GmbH under number DSM 23882, respectively, on August 5, 2010.

### Culture-morphological characteristics

Culture-morphological characteristics were determined after cultivation in MRS agar and broth (Oxoid) media.

MRS broth during 24-48 hours in anaerobic environment was suitable for the cultivation of *L. gasseri* MCC2, thereafter a uniformly turbid growth was seen in the broth. After cultivating for 48 hours on MRS agar culture media at 37°C in anaerobic environment (CO₂/H₂/N₂:5/5/90) the colonies of *L*. *gasseri* MCC2 are with a diameter of 1.5-2 mm, off-white, rough and with irregular margin. The cells are short to coccoid rods with medium thickness. The optimum growth temperature is 37°C; the strain multiplies also at 45°C. The pH of optimum growth environment is 6.5. *L. gasseri* MCC2 is catalase- and oxidase-negative, obligately homo-fermentative (OHOL), does not hydrolyze arginine and does not produce gas during glucose fermentation.

*L. gasseri* MCC2 was identified on the basis of API 50CHL System (bioMérieux, France) test-kit as *L. delbrueckii ssp. delbrueckii* (coincidence with type strain: good, ID %-94.6, T index 0.35). Molecular identification of *L. gasseri* MCC2 on the basis of 16S rRNA sequence showed homology with type strain *L. gasseri* IDCC 3102 (BLAST http://www.ncbi.nlm.nih.gov/guide/sequence-analysis/).

Carbohydrate fermentation profile of *L. gasseri* MCC2 was the following on the basis of API CHL 50. The strain ferments: lactose, saccharose, trechalose and esculine.
According to API ZYM (bioMérieux, France) test-kit *L. gasseri* MCC2 has got leucin arylamidase, valine arylamidase, α-glucosidase, and β-glucosidase activity.

### Safety

The origin of the microbe strains from the digestive tract of a healthy child proves their GRAS *(generally recognised as safe)* status or the microbe strains are safe for humans and suitable for oral administration. Also the species of lactobacilli *L. gasseri* have been entered in the list of taxonomic units with QPS (*Qualified Presumption of Safety*) status by EFSA (EFSA. Introduction of a Qualified Presumption of Safety (QPS) approach for assessment of selected microorganisms referred to EFSA. EFSA J 2007; 587, 1-16).

### Haemolytic activity

Methodology: The haemolytic activity of *L. gasseri* MCC2 was determined after incubation in microaerobic environment on blood agar plates (Blood Agar Base nr 2, Oxoid +7% human or equine blood).
Result. The strain *L. gasseri* MCC2 had no haemolytic activity.

### Resistance to antibiotics

Methodology: The susceptibility of *L. gasseri* MCC2 to antibiotics was tested with the help of E-test (AB Biodisk, Solna). The minimum inhibitory concentration was determined according to the epidemiological breakpoints recommended by the European Commission.

**Table 1. Antibacterial susceptibility of L. plantarum MCC1 DSM 23881 and L. gasseri MCC2 DSM 23882 (MIC, mg/L)**

| | MIC* values (µgmL) | | | | |
|---|---|---|---|---|---|
| Antibiotic | *L .plantarum MCC1* | *L. gasseri* MCC2 | *Lactobacillus* spp** | *L. plantarum^{#}* | OHOL^{#} |
| Ampicillin | 0.094 | 0.031 | 2 | 2 | 1 |
| Gentamicin | 1.5 | 2 | 1 | 16 | 16 |
| Streptomycin | 16 | ND | 16 | n.r.** | 16 |
| Erythromycin | 0.125 | 0.047 | 4 | 1 | 1 |
| Clindamycin | 0.064 | ND | n.r. | 1 | 1 |
| Tetracycline | 2 | 0.5 | 16 | 32 | 4 |
| Chloramphenicol | 1.5 | 0.75 | 16 | 8 | 4 |
| Quinupristin/ dalfopristin | 1 | ND | 4 | 4 | 4 |
| Cefoxitin | 256 | 1.5 | 4 | n.r. | n.r. |
| Cefuroxim | 0.5 | 0.19 | - | n.r. | n.r. |
| Imipenem | ND | 0.047 | - | n.r. | n.r. |
| Ciprofloxacin | 0.38 | >32 | 4 | n.r. | n.r. |
| Rifampin | ND | 0.064 | 32 | n.r. | n.r. |
| Trim/sulfam | ND | >32 | 32 | n.r. | n.r. |
| Vancomycin | 256 | 0,75 | 4 | n.r. | 2 |

| | | | | | |
|---|---|---|---|---|---|
| *MIC - minimum inhibitory concentration, breakpoint for resistance, OHOL - Obligately homofermentative lactobacilli, n.r. - not required# ** European Commission (EC), 2002. Opinion of the Scientific Committee on Animal Nutrition on the criteria for assessing the safety of microorganisms resistant to antibiotics of human clinical and veterinary importance. European Commission, Health and Consumer Protection Directorate General, Directorate C, Scientific Opinions, Brussels, Belgium. http://ec.europa.eu/food/fs/sc/scan/out64 en.pdf # FEEDAP Panel EFSA Technical guidance prepared by the Panel on Additives and Products or Substances used in Animal Feed (FEEDAP) on the update of the criteria used in the assessment of bacterial resistance to antibiotics of human or veterinary importance. The EFSA Journal (2008) 732, 1-15 | | | | | |

The strain *L.gasseri* MCC2 did not have resistance to tested antibiotics.

### Functional properties of L. gasseri MCC2

### Profile of metabolites

Methodology: The metabolite profile has been established with gas chromatography (HP 6890) following 24 h and 48 h of incubation in microaerobic growth environment (Table 2). Lactobacilly were grown for 48 hours on MRS agar culture media in 10% CO₂ environment, then suspension was prepared into 0.9% NaCl solution with a density 10⁹ microbes/mL according to McFarland, and 1.0 mL of obtained suspension was inoculated into 9.0 mL of MRS liquid culture media. The amount of metabolites mmol/L was determined using capillary column HP-INNOWax (15 m x 0.25 mm; 0.15 µm). Column temperature program 60°C 1 min, 20°C/min 120°C 10 min; detector (FID) 350°C.

**Table 2. Concentration of acetic acid, lactic acid and succinic acid (mmol/L) in MRS culture media following 24 h and 48 h microaerobic cultivation of L. plantarum MCC1 and L. gasseri MCC2 in MRS culture media.**

| Species | Acetic acid (mmol/L) | | Lactic acid (mmol/L) | | Succinic acid (mmol/L) | |
|---|---|---|---|---|---|---|
| | 24h | 48h | 24h | 48h | 24h | 48h |
| ***L**. plantarum* MCC1 | 0.9 | 1.3 | 100.0 | 137.2 | 0.4 | 0.5 |
| *L. gasseri* MCC2 | 0.7 | 0.7 | 39.7 | 48.4 | 0.3 | 0.4 |

### Determination of L. gasseri MCC2 aggregation and milk coagulation ability

Methodology: Milk coagulation ability. The ability to coagulate milk was studied after incubating the strain for 48 h in microaerobic conditions in UHT fat-free milk at 37°C.

Coagulation ability was assessed according to the following scheme:
1. Strong (+) - milk coagulates during 24 h,
2. Slow (+/-) - milk coagulates during 48 h,
3. Absent (-) - milk doesn't coagulate.

Autoaggregation. To assess the ability of autoaggregation the strain previously pre-cultivated for 24 h on MRS agar culture media were suspended in physiological solution. The obtained suspension was kept for 15 minutes at room temperature. Aggregation ability was assessed according to the following scheme: 0 - absent, suspension homogenously turbid; 1 - some flakes in the solution; 2 - lots of flakes in the suspension, slight deposit on the bottom of the test tube; 3 - intensive, no suspension develops, deposit on the bottom, solution almost transparent.

*L. plantarum* MCC1 and *L. gasseri* MCC2 coagulated milk during 24 hours, had strong autoaggregative properties (Table 3).

**Table 3. Aggregation, general proteolysis and milk coagulation ability of the strains**

| Strain | Autoaggregation | Milk coagulation |
|---|---|---|
| *L.plantarum* MCC1 | 3 | + |
| *L.gasseri* MCC2 | 3 | + |

### Fermentation ability of prebiotics

To assess the fermentation of Tagatose (Arla Food Ingredients), Raftilose P95 (Beneo Orafti), Raftilose Synergy 1 (Beneo Orafti) and Raftilose L60/75 (Orafti), 0.05 mL of 48 h old culture of the tested *Lactobacillus sp.* was inoculated into MRS culture media, which contained 0.5% of sorbite, tagatose or melecitose instead of glucose. Chlorophenol red (0.004%) was added to the culture media as an indicator. Fermentation was assessed on the basis of colour change (from red to yellow) after 48 h.

**Table 4. The ability to use prebiotics by L. plantarum MCC1 and L. gasseri MCC2**

| | *L.plantarum* MCC1 | *L.gasseri* MCC2 |
|---|---|---|
| Tagatose | - | + |
| L60/75 | + | + |
| Raftilose Synergy 1 | + | - |
| Raftilose P95 | + | + |

### Antimicrobial activity against pathogens

Methodology: For the assessment of antimicrobial properties streak-line method was used (Hütt P, Shchepetova J, Loivukene K, Kullisaar T, Mikelsaar M. Antagonistic activity of probiotic lactobacilli and bifidobacteria against entero- and uropathogens. J Appl Microbiol. 2006; 100(6):1324-32).
To determine the inhibition of pathogens the growth-free zone was measured in mm. Similarly to Hütt et al (2006) the arithmetic mean and standard error were calculated on the basis of the results of the used sample and the antagonistic activity of the strains (mm) during incubation at 37°C was assessed as follows: - weak <9.7; medium 9.7-12.2; strong >12.2. All tests were repeated in parallel at least three times.

**Table 5. Antimicrobial activity of L. plantarum MCC1 and L. gasseri MCC2 against pathogens with streak-line method during cultivation in anaerobic and microaerobic environments (inhibition of pathogen growth, mm)**

| Pathogen | *L. plantarum* MCC1 | | *L. gasseri* MCC2 | |
|---|---|---|---|---|
| | Anaerobic | Microaerobic | Anaerobic | Microaerobic |
| *Shigella sonnei* | 20.3±2.3 | 20.3±2.1 | 9.5±2.9 | 7.7±1.5 |
| *Staphylococcus aureus* | 15.7±2.9 | 15.0±1.0 | 3±3.2 | 2.3±2.5 |
| *Escherichia coli* | 17.3±2.5 | 18.0±1.0 | 6.3±3.4 | 6.3±1.2 |
| *Enterococcus faecalis* | 16.7±2.1 | 14.7±2.7 | 4.5±2.9 | 2.0±1.0 |
| *Salmonella enterica* serovar Typhimurium | 18.8±2.1 | 17.8±1.8 | 7.15±1.6 | 4.3±2.1 |
| *S. enteritidis* | nd | 7.1±2.8 | nd | 2.2 ±2.6 |
| *Listeria monocytogenes* | nd | 12.8±1.7 | nd | 2.8±1.0 |
| *Yersinia enterocolitica* | nd | 12.8±1.0 | nd | 3.5±2.6 |

| | | | | |
|---|---|---|---|---|
| Weak <9.7; medium 9.7-12.2; strong >12.2; nd - not determined | | | | |

*L.gasseri* MCC2 turned out to be a weak antagonist in both environments.

### General proteolytic properties

General protelytic properties of *L. gasseri* MCC2 were studied on SMA culture media (skim milk-agar media) and on Ca-caseinate agar, streaking the strains previously pregrown during 48 h in MRS broth onto the test culture media. These were incubated during 7 days in microaerobic environment at 37°C. The strength of proteolysis was assessed (6 tests, arithmetic mean) on the basis of rating system: 4 - strong, 3 - medium, 2 - weak, 1 - very weak, 0 - absent.

Results: *L. gasseri* MCC2 has sufficiently good general proteolytic effect (in a 4-point system *L. gasseri* MCC2 has two points).

### Bioselection on the basis of more specific proteolytic ability (mass-spectrometric and RP-HPLC technological tests to identify the extent of proteolysis of α-S1-casein and β-casein)

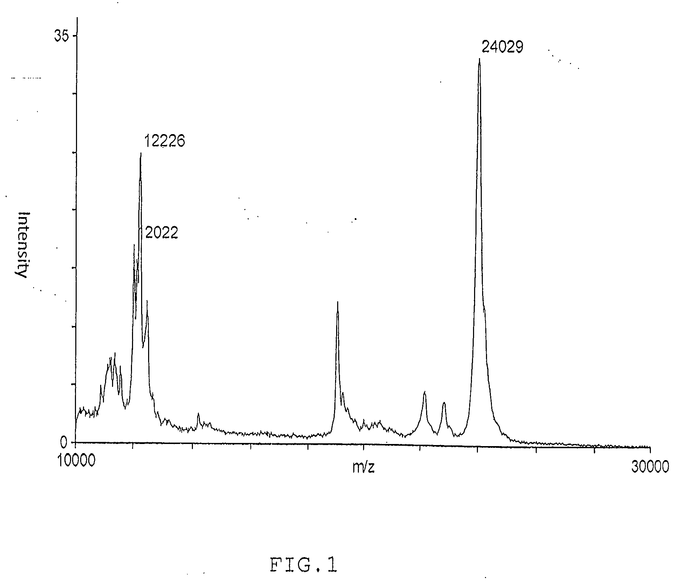

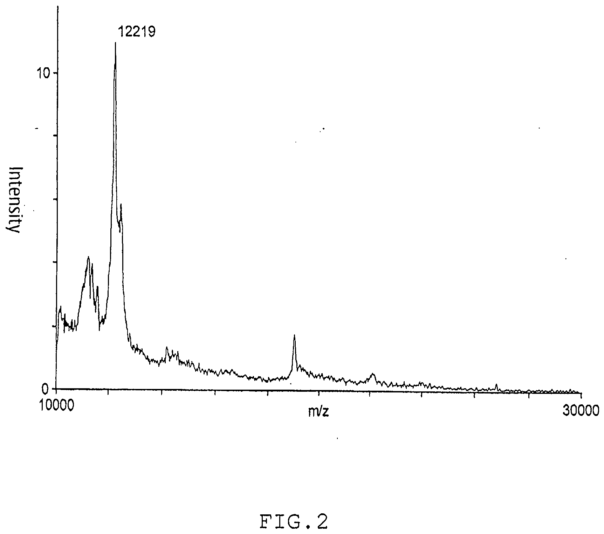

It was not known for *L. gasseri* MCC2 how it hydrolyzes different milk proteins. So at first the changes in molecular mass spectra occurring during hydrolysis were ascertained using individual pure milk proteins (β-lactoglobulin, α-S1 casein, β-casein) to analyse the protein specter changes of unskimmed milk later. The molecular masses of milk protein hydrolysis products were determined on the MALDI-TOF *(Matrix-assisted laser desorption*/*ionization time-of-flight)* mass-spectrometer (Marsilio, R. Catinella, S. Seraglia R. Traldi P. (1995) Matrix-Assisted Laser-Desorption Ionization Mass-Spectrometry for the Rapid Evaluation of Thermal-Damage in Milk. Rapid Communications in Mass Spectrometry, 9, 550-552). A suspension with a density of 10⁹ CFU/mL according to McFarland 4 Standard was prepared from the cultures into KCl physiological solution. 10 µl of milk protein fraction was added to obtained suspension. The survival of the strain in the solutions of these fractions was determined with streaking the dilution series before and after the test. The strains were incubated in corresponding protein fraction environment for 2-6 days at 37°C. Then the samples were centrifuged and protein profile was determined on mass-spectrometer (*Voyager DE Pro, Applied Biosystems)* Figure 1 and Figure 2). It was found that the strain that is the object of the invention is able to adjust metabolism to nutrient-poor environment. For example in the test the only nutrient source was either α-S1 casein, β-lactoglobulin, β-casein or the substances obtained from the strain cells themselves. The strain was detectable at the end of the test in all three protein fraction environments (α-S1 casein, β-lactoglobulin, β-casein).

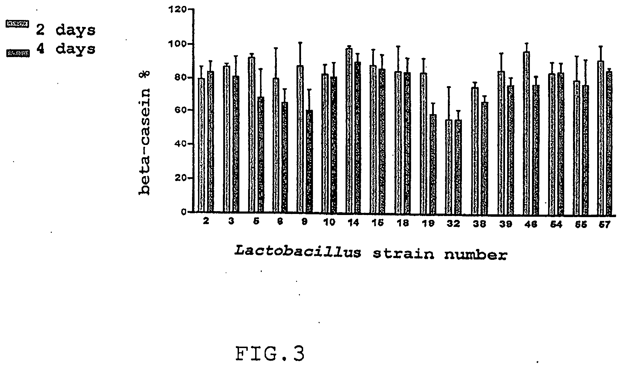

Then the proteolytic properties of the strain was investigated in marketed milk with 2.5% fat content with the help of RP-HPLC *(Reversed-Phase High-Performance Liquid Chromatography)* method. The extent of proteolysis of α-S1 casein and β-casein was measured on the 2nd and 4th day of incubation (Figure 3). For this the RP-HPLC chromatogram peak area corresponding to investigated milk protein was compared to the start moment (control, i.e. milk before the addition of lactobacilli, was equated with 100%) and the percentage amount of remaining milk protein was calculated. In addition experiments were carried out with longer than 21-day incubation times. Also combinations of the strains that are the objects of this invention were investigated to improve proteolytic efficacy and proteolytic activity also with whey (to reduce the amount of allergic caseins) in case of prediluted milk. The preparation of milk samples for RP-HPLC analysis and the conditions of chromatographing have been developed along the lines of methodology described by Bobe et al. (Bobe G, Beitz DC, Freeman AE, Lindberg GL. (1998) Separation and Quantification of Bovine Milk Proteins By reversed-Phase High-Performance Liquid Chromatography. J. Agric. Food Chem., 46, 458-463). Marketed milk with 2.5% fat content where 10⁷ CFU/mL of investigated strain was added and incubated at 37°C, was compared to milk without the strain. High-performance liquid chromatograph (HPLC) (Hewlett Packard 1100) and reverse phase column Zorbax 300SB-C18 filled with porous silica were used for the separation of milk proteins. The mobile phase was an eluting system with a changing gradient, consisting of: Solution A - acetonitrile, water, TFA in ratio 900:100:1 (v/v/v); Solution B - acetonitrile, water, TFA in ratio 100:900:1 (v/v/v). The gradient starts with solution B and the proportion of solution A starts to increase immediately after the sample is injected into the system. During separation phase, when the milk protein separation occurs, the content of solution A increases by 0.47% per minute. The obtained fractions were identified by comparing the retention times with the retention times of the main milk protein standards and determining the molecular mass with MALDI-TOF mass-spectrometer. Milk proteins elute in the next sequence: κ-CN, α_{S2}-CN, α_{S1}-CN, β-CN, α-LA and β-LG. *L. gasseri* MCC2 had appropriate effects in relation to α-S1 casein and β-casein (see summary data in Figure 1 and Figure 2), the amount of proteins dominating in the children's allergy reply to cow's milk, α-S1 casein and β-casein, was reduced on the 2^{nd} day. On the basis of RP-HPLC analysis the best directed partial proteolyzer was *L. gasseri* MCC2 (Figure 3). Combining the aforementioned lactobacilli between themselves, the proteolytic effect in milk was somewhat reduced compared to the individual use of the same strains. *L.plantarum* MCC1 was also incubated for 4 days in a mixture consisting of 40% milk and 60% whey: the content of α-casein was on the fourth day 81% and the content of β-casein 83% compared to the starting moment.
The set objective was achieved: the strain *L. gasseri* MCC2 and its combination with *L.plantarum* MCC1 reduced the amount of α-S1 casein and/or β-casein by 20-40% (i.e. directed moderate hydrolysis took place).

*L. gasseri* MCC2 hydrolyzes the proteins causing milk allergy and it has a good general proteolytic activity. The use of *L*. *gasseri* MCC2 separately or together with *L.plantarum* MCC1 reduces milk allergy and therefore they can be used to produce a milk allergy reducing food product and dietary supplement with healthy properties.

### Bioselection on the basis of conjugated linoleic acid (CLA) production

CLA stands for a group of 18-carbon fatty acid, linoleic acid (LA, *cis-9, cis-12-18:2*) isomers, where the double bonds are conjugated. CLA is formed naturally during the processes of biohydrogenation and oxidation. CLA is thought to have anti-adipogenous, anti-cancerogenous, anti-diabetogenous and anti-inflammatory effect, regulatory effect on immune system, production of cytokines and immunoglobulins and an ability to modulate the expression of certain genes directly or indirectly through specific transcription factors (Wahle, K. W. J., Heys, S. D. and Rotondo, D. (2004). Conjugated linoleic acids: are they beneficial or detrimental to health? Prog. Lipid Res. 43: 553-587). As several lactic acid bacteria are able to change linoleic acid into conjugated linoleic acid, then it would be possible to increase the CLA content in fermented milk products (Sieber, R., Collomb, M., Aeschlimann, A., Jelen, P. and Eyer, H. (2004). Impact of microbial cultures on CLA in dairy products- a review. Int. Dairy J. 14: 1-15). For this it should be tested, which lactic acid bacteria are CLA producers. It is known that free fatty acids occurring in the growth environment of microbes have bacteriostatic effect, influencing microbe growth and cell membrane permeability. The strength of the effect depends on the fatty acid. Long-chain fatty acids with higher level of saturation have stronger antimicrobial effect. The stereochemistry of double bonds also influences the antimicrobial activity: a fatty acid with cis-configuration is a stronger inhibitor than trans-form (Kankaanpää, P. E., Salminen, S. J., Isolauri, E., Lee, Y. K. (2001). The influence of polyunsaturated fatty acids on probiotic growth and adhesion. FEMS Microbiol. Lett. 194: 149-153). The microbes have an established defence mechanism for counteracting a compound inhibiting its vital functions: polyunsaturated fatty acids are isomerized to less inhibiting variants, for example linoleic acid (LA) with stronger bacteriostatic effect is converted to CLA (Sieber, R., Collomb, M., Aeschlimann, A., Jelen, P. and Eyer, H. (2004). Impact of microbial cultures on conjugated linoleic acid in dairy products- a review. Int. Dairy J. 14: 1-15). Most of the emerged CLA stays in the environment; one part is added to the lipids of cell membrane. It has been found that CLA may in small quantities be located in the microbial cell. The ability to conjugate LA and other fatty acids (oleic acid, ricinolic acid) has been found in the representatives of several microbial genera, including *Lactobacillus sp.* The ability of the strains to produce CLA on the basis of linoleic acid added to the growth environment (MRS, skim milk) was assessed quantitatively using CLA determination method developed by us. It became clear that *L. plantarum* MCC1 and *L. gasseri* MCC2 that had the needed purposeful proteolytic effect produced CLA significantly (Table 7). At the same time these strains were not practically susceptible to the bacteriostatic effect of LA.

### Bioselection on the basis of oxidation resistance (antioxidativity)

It was investigated if the strain *L. gasseri* MCC2 that has specific limited proteolytic properties, also CLA-productivity, has physiologically significant oxidation resistance. Oxidation resistance should be determined with at least two methods.

Linoleic acid test (LA-test) which makes possible to determine the ability of cell suspensions of lactobacilli to inhibit peroxidation of linoleic acid, was used as the first method.

A commercial test (Total Antioxidant Status - TAS, Randox Laboratories Ltd., UK) based on the inhibition of the formation of ferryl metmyoglobin radical generated by hydrogen peroxide by cell suspension of lactobacilli, was used as the second method (Rice-Evans and Miller 1994). TAS value was reported in trolox (soluble form of vitamin E) units (mmol/L). Both methods have been published (Kullisaar T, Zilmer M, Mikelsaar M, Vihalemm T, Annuk H, Kairane C, Kilk A. (2002). Two antioxidative lactobacilli strains as promising probiotics. Int. J. Food Microbiol. 72, 215-224; Kullisaar, T., Songisepp, E., Mikelsaar, M., Zilmer, K., Vihalemm, T., Zilmer, M. (2003) Antioxidative probiotic fermented goats milk decreases oxidative stress-mediated atherogenicity in human subjects. British Journal of Nutrition 90, 449-456).

For the determination of TAA and TAS the strains were incubated in MRS broth (Oxoid) for 24 h at 37°C. The microbial cells were centrifuged at 4°C and 1500 rpm for 10 minutes, washed with isotonic saline (4°C) and suspended in 1.15% KCl (Sigma, USA). The suspension density at OD₂₆₀ 1.1 was 10⁹ microbial cells/mL. *L. plantarum* MCC1 and *L. gasseri* MCC2 have physiologically significant total antioxidativity (Table 6).

**Table 6. Oxidation resistance with TAA and TAS methods (n=7-9; M±SD)**

| Strain | TAA (%) | TAS (mmol/l) |
|---|---|---|
| *L.plantarum* Inducia DSM 21379 | 26±12 | 0.13±0.04 |
| *L. plantarum* MCC1 DSM 23881 | 20±8 | 0.05±0.02 |
| *L.gasseri* MCC2 DSM 23882 | 25±6 | 0.03±0.03 |
| *L.plantarum* Tensia DSM 21380 | 22±5 | 0.05±0.02 |

### Bioselection on the basis of nitrogen oxide and hydrogen peroxide production ability

To obtain purposeful information, the preselected strains (specific limited proteolytic properties, CLA production, antioxidativity) were also investigated in respect to NO and H₂O₂ production. According to the literature there are no previous studies about concurrent production of NO and H₂O₂ by microbial strains. The measurements were carried out with live cells using electrochemical measuring (*Apollo* 4000 free radical analyzer WPI, Berlin, Germany), using ISO-HPO2 and ISO-NOP type electrodes. The electrodes were placed into culture grown in MRS broth (Oxoid), the signals were recorded concurrently during 5-7 minutes and mean signal strength during the measuring time was calculated. Each experiment point was measured as 4 independent parallels and each parallel was measured twice. The NO and H₂O₂ concentrations in the investigated sample were determined by comparison of sample signal with the standard curve.

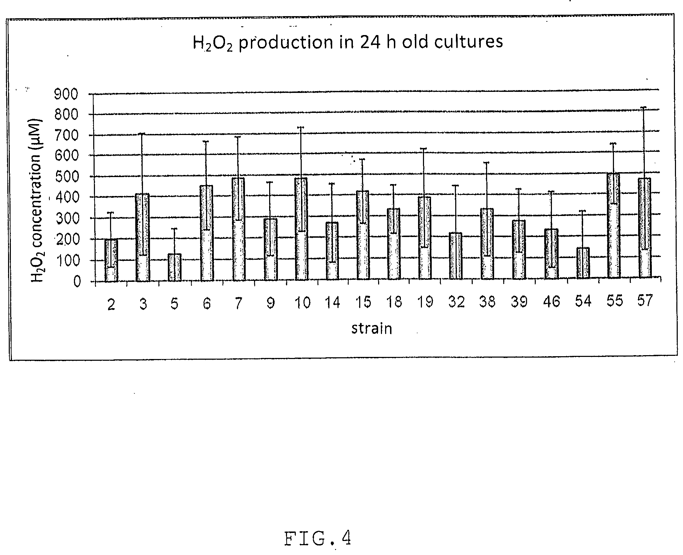

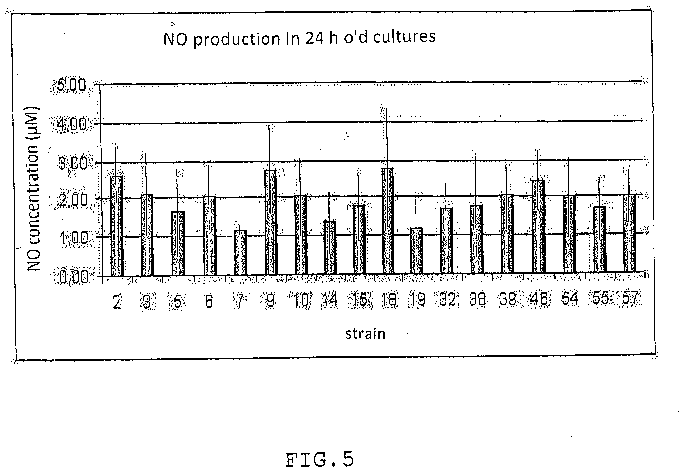

*L. gasseri* MCC2 has significant ability to produce NO and hydrogen peroxide (Figures 4 and 5).

Therefore the strain *L. gasseri* MCC2 is suitable as antioxidant additives for the production of food product and dietary supplement.
Table 7 shows the summary of functional properties of abovementioned strains and their combination.

**Table 7. Summary of functional properties of the strains L. plantarum MCC1 and L. gasseri MCC2 and their combination**

| FUNCTIONAL PROPERTIES | | MCC1 | MCC2 |
|---|---|---|---|
| 1 | *In vitro* antagonism:# | 2 | 1 |
| 2 | Production of bactericidal H₂O₂ 1 | 3 | 2 |
| 3 | Production of bactericidal and relaxing factor NO 2 | 3 | 2 |
| 4 | CLA production*** | | |
| | MRS | 3 | 2 |
| | Skim milk | 3 | 1 |
| 5 | General proteolytic effect | 3 | 2 |
| | Oxidation resistance & | | |
| | TAA | 3 | 3 |
| | TAS | 1 | 1 |
| 7 | Fermentation of prebiotics 4 | 3 | 1 |
| 8 | Production level of biogenic amines 3 | 3 | 3 |
| 9 | Proteolyticity of caseins 5 | 2 | 3 |
| | Sum of positive scores | 29 | 21 |
| SAFETY AND POSITIVE EFFECTS | | MCC1 | MCC1+MCC2 |
| 1 | Haemolytic activity *in vitro* | 0 | |
| 2 | Animal test (NIH line mice) | | |
| | Translocation of strain | 0 | |
| | Histological changes | 0 | |
| 3 | Safety studies in volunteers, 2 weeks | n = 10 | n = 9 |
| | Lower abdomen pain | 0/30SC 30 | 0/27SC 27 |
| | Flatulence | 0/30SC 30 | 0/27SC 27 |
| | Summary score | 3 | 3 |
| 4 | BMI, blood pressure, blood glucose, blood lipids, cholesterol, lipoproteins, liver function, inflammation and OxS markers did not differ from endemic reference values | 3 | 3 |
| 5 | Statistically true positive changes in systemic inflammation and oxidative stress markers | 3 MPO 3 Isoprostan es | 3 hsCRP, 3 IL-10 3 Isoprostan es |
| 6 | Significant other trends (number of other markers) | 3 (oxLDL, B2GPI-LDL,sdLDL) | 5 (IgE, BP, oxLDL, b2GPI-LDL,sdLDL) |
| Sum of positive scores | | 15 | 20 |

| | | | |
|---|---|---|---|
| #SCORE 3 >= 20 mm; SCORE 2 = 15-20 mm; SCORE 1 = < 15 mm, ## SCORE 3 - no complaints; SCORE 2 - some complaints; SCORE 1 - changes, complaints, ¹ SCORE 3 - 400-500; SCORE 2- 300 -399; SCORE 1 - 100-299 units, ² SCORE 3 - 2.0 - 3.0; SCORE 2 - 1.0 - 1.99; SCORE 1- <1.0 units, ⁴ SCORE 3 - Inulin, its derivatives, tagatose SCORE 2 - only inulin or tagatose - no inulin or tagatose, ⁵ SCORE 3 - 30-45%, SCORE 2 - 15-30%, SCORE 1 - < 15%, ³ SCORE 3 - only traces; SCORE 2 - < 10; SCORE 1- 11-30 units (during long-term effect), ^{&} SCORE 3 TAA > 20, TAS >0.1; SCORE 2 - TAA >15, TAS 0.05-0.09; SCORE 1 - TAA <15, TAS < 0.05 units, *** SCORE: CLA production 3 - 40-31, SCORE 2 30 - 20-15, SCORE 1 14 -10 units | | | |

Afore-cited described the microorganism strain being the object of the invention.

The next object of the invention is composition containing strain *L. gasseri* MCC2.

In the composition the strain *L. gasseri* MCC2 content is in the range of 0.8-1.2 volume percent and the germ count is 0.5x10⁷-1x10⁸ CFU/mL.

Preferably the content of the strain *L. gasseri* MCC2 is 1.0 volume percent, and the germ count of is 0.8x10⁷-1.2x10⁸ CFU/mL.

If the composition contains strain *L.plantarum* MCC1 and *L. gasseri* MCC2 the content of both is in the range of 0.4-0.6 volume percent, whereat the germ count of *L. plantarum* MCC1 strain is in the range 0.2x10⁸-2.4x10⁸ CFU/mL and the germ count of *L. gasseri* MCC2 strain is 0.4x10⁷-0.6x10⁸ CFU/mL. When both strains are used the content of both strains is preferably at least 0.5 volume percent, whereat the germ count of *L. plantarum* MCC1 strain is in the range 0.25x10⁸-2x10⁸ CFU/mL and the germ count of *L. gasseri* MCC2 strain is 0.5x10⁷-0.5x10⁸ CFU/mL.

The aforementioned strain *L. gasseri* MCC2 may be live or killed.

The next objects of the invention is the use of *L. gasseri* MCC2
- as antioxidant proteolytic ingredient for the production of food product and/or dietary supplement,
- for the production of hypoallergenic food product and/or dietary supplement reducing milk allergy,
- for the production of food product and/or dietary supplement reducing lower urinary tract irritation symptoms accompanying benign prostatic hyperplasia and oxidative stress and inflammation associated with these.

The next object of the invention is the method for the production of food product and/or dietary supplement reducing milk allergy and lower urinary tract irritation symptoms and oxidative stress and inflammation associated with these.

The method according to the invention includes the next stages:
A) The initial mixture containing milk protein is warmed to 37+2°C;
B) *L. gasseri* MCC2 alone or together with *L. plantarum* MCC1 is added. *L. gasseri* MCC2 will be added up to 1.2 volume percent, preferably 1 volume percent with germ count 0.8x10⁷-1.2x10⁸ CFU/mL. When both abovementioned strains are used together, they will be added up to 1.2 volume percent, preferably 1 volume percent, whereas *L. plantarum* MCC1 will be added 0.25x10⁸-2x10⁸ CFU/mL and *L. gasseri* MCC2 0.5x10⁷-0.5x10⁸ CFU/mL. The end-pH of fermenting is 4.2±0.25.
C) The mixture is fermented by *L. gasseri* MCC2 or by *L.plantarum* MCC1 + *L.gasseri* MCC2 at 37+2°C at least 18-26 hours.
D) The mixture is pasteurized in the range of 80-85°C (preferably 82°C±2) for 30-35 minutes (preferably 30 minutes) and cooled down to temperature 20-30°C.
E) The obtained mixture is flavoured with additives and cooled down to temperature 2-6°C.
F) The obtained mixture is used for the production of food product and/or dietary supplement. In case of food product the mixture is bottled, stored (at 2-6°C) and all required analyses are performed (determination of oxidation resistance etc.). The mixture may also be used in powder (capsules, lozenges, tablets, powder sachets etc.) or liquid (ampoules) form for the production of marketed dietary supplement.

During the preparation of the dietary supplement in powder form water is removed from the mixture, for example by lyophilization (freeze-drying), spray-drying, mill-drying or other known method (until the powder consistency is obtained). During the preparation of liquid dietary supplement water is removed from the product partially until the desired consistency is obtained). The dietary supplement may be prepared with appropriate additive (e.g. antioxidants, sweeteners, prebiotics) or without.

In the aforementioned method the source of milk protein is milk, milk powder, milk protein concentrate, whey or other milk protein source. Fruit or berry juice, -concentrate, syrup or juice drink or fruit or berry jam, preferably sea buckthorn, blueberry or raspberry juice or other juice, syrup, concentrate are suitable additives.

### DESCRIPTION OF THE DRAWINGS

Figure 1 - MALDI-TOF mass-spectrometric analysis (illustrative typical experiment). Beta-casein 30 minutes after mixing with strain *L. gasseri* MCC2.
Figure 2 - MALDI-TOF mass-spectrometric analysis (illustrative typical experiment). After 48 h incubation with the strain *L. gasseri* MCC2 the mass specter peak of beta-casein 24,0 kDa was substantially eliminated.
Figure 3 - Effect of strains *L. plantarum* MCC1 and *L. gasseri* MCC2 in case of 48 and 96-hour incubation on the level of beta-casein (the basal level was 100%, on the basis of MALDI-TOF mass-spectrometric and RP-HPLC analysis data).
Figure 4 - NO production by strains *L. plantarum* MCC1 and *L. gasseri* MCC2.
Figure 5 - H₂O₂ production by strains *L. plantarum* MCC1 and *L. gasseri* MCC2.
Figure 6 - The scheme of the production of food product and dietary supplement.

### DESCRIPTION OF THE EMBODIMENTS

Example 1. Method for production of milk allergy responses and urinary tract irritation symptoms and associated oxidative stress and inflammation reducing food product and dietary supplement with abovementioned healthy properties, containing microorganisms *L. plantarum* MCC1 or *L. gasseri* MCC2 or their combination.

Below is presented the preferable embodiment.

For the production of food product and/or dietary supplement reducing milk allergy responses and urinary tract complaints microbial cultures were used that were pre-grown in fermenter during 24 h and then lyophilized.

For the production of food product the lyophilized microbial cultures *L. plantarum* MCC1 and/or *L. gasseri* MCC2 were activated in small amount of warm (at least 20°C) whey, milk or solution made from milk powder/milk protein concentrate or other milk protein source for at least 4 hours.

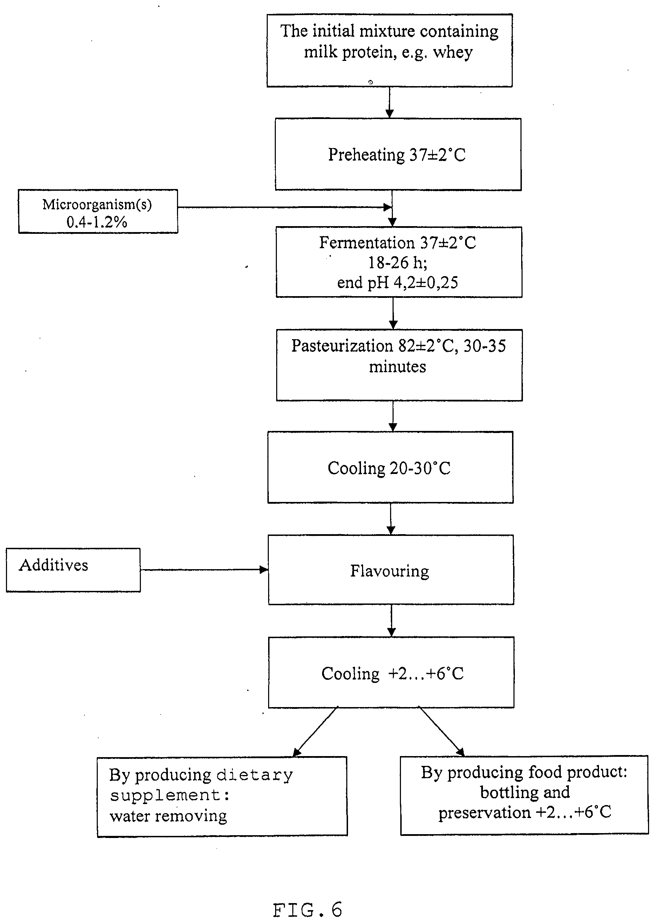

Method for production of food product or dietary supplement with healthy properties included the next stages (see scheme in figure 6):
a) The initial mixture containing milk protein, whey obtained from cheese industry (chemical-physical properties in table 8), in which fat phase was reduced by separation and cheese dust was removed, was warmed to fermentation temperature 37±2°C;
b) 1 volume percent of strain *L. plantarum* MCC1 (with germ count 0.5 x10⁸-4x10⁸ CFU/mL) or strain *L*. *gasseri* MCC2 (with germ count 1x10⁷-1x10⁸ CFU/mL) or both together (both 0.5 volume percent containing live cells of strain *L*. *plantarum* MCC1 with a density in a range 0.25-2x10⁸ CFU/mL and live cells of strain *L. gasseri* MCC2 with a density in a range 0.5x10⁷-0.5x10⁸ CFU/mL.

**Table 8. Chemical-physical properties of whey**

| Ingredient/parameter | Content |
|---|---|
| Fat (%) | < 0.1 |
| Protein (%) | 0.66±0.10 |
| Lactose (%) | 4.07±0.59 |
| Dry substance (%) | 5.37±0.27 |
| Density (g/cm³) | 1.0199±0.0002 |
| pH | 6.14±0.41 |

c) The mixture was fermented for 18-26 h at temperature 37±2°C, during which a partial hydrolysis of milk protein took place due to the effect of strain (s) *L. plantarum* MCC1 and/or *L.gasseri* MCC2. The fermentation lasted until acidity reached the value of pH 4.20 ± 0.25;
d) To inactivate (kill) the strain(s) *L. plantarum* MCC1 and/or *L. gasseri* MCC2 the mixture was pasteurized at 82±2° C for 30-35 minutes and cooled to temperature 20-30°C;
e) The obtained mixture was flavoured with selected additives, which could be fruit or berry juice, - concentrate, -syrup, juice drink or fruit or berry jam. For example 16-17 volume percent of sea-buckthorn jam or 4.7-5 volume percent of concentrated cherry juice drink and sea-buckthorn juice and 9.8-10.2 volume percent of raspberry-blueberry concentrated juice drink were used.
f) The obtained mixture was cooled to 2-6°C, the food product was bottled, organoleptic and oxidation resistance analysis performed and stored (2-6°C) and clinical studies were performed (see below).

To produce a powder dietary supplement water was removed from the obtained mixture using methods known in food processing technology (for example with the help of lyophilization or drying (spray-drying, mill drying etc.)). Preferably a one-stage spray drier (air inflow temperature 160°C and air outflow temperature 80°C) was used to produce a powder dietary supplement.
To the mixture produced by the abovementioned way a suitable filling agent was added that had a melting point higher than lactose, for example maltodextrin (in 1:1 ratio with whey dry substance).
Water, sugar (lactose) and salt separation with ultrafiltration was used as another choice, which was followed by concentrating with vacuum machine and drying (spray-drying), and so a powder dietary supplement with low lactose content and high protein content was obtained.
As a third choice concentrating with vacuum device followed by spray-drying was used for water removal. The final result was a powder dietary supplement containing lactose.

Table 9. Chemical-physical properties of food product (whey drink) containing strains MCC1+MCC2, flavoured with sea-buckthorn jam, and of dietary supplement containing strains MCC1+MCC2, flavoured with sea-buckthorn jam

| Ingredient/parameter | Content | |
|---|---|---|
| | Whey drink | Dietary supplement |
| Calculated energetic value of food | | |
| kcal/100g | 66 | 390 |
| kJ/100g | 219 | 1654 |
| Fat (%) | 0.41 | 3.46 |
| Protein (%) | 0.90 | 5.96 |
| Ash (%) | 0.46 | 3.4 1 |
| Dry substance (%) | 16.36 | 96.58 |
| Calculated carbohydrate content (%) | 14.6 | 83.8 |
| Glucose (%) | <0.5 (0.1) | 1.1 |
| Fructose | <0.5 (0.02) | <0.5 (0.2) |
| Saccharose | <0.3 (0) | <0.3 (0.1) |

Food products FP-I - FP-V containing the microorganisms being the objects of the invention and additives were produced in the abovementioned way as follows:
Food product I (or FP-I): Milk protein containing mixture + *L. plantarum* MCC1 + *L. gasseri* MCC2; additive sea-buckthorn juice (BT);
Food product II (or FP-II): Milk protein containing mixture + *L. plantarum* MCC1; additive raspberry-blueberry concentrated juice drink (RB).
Food product III (or FP-III): Milk protein containing mixture *+ L. plantarum* MCC1 + *L.gasseri* MCC2; additive blackcurrant-raspberry concentrated juice drink (BR).
Food product IV (or FP-IV): Milk protein containing mixture + *L. plantarum* MCC1; additive blueberry concentrate (B).
Food product V (or FP-V): Milk protein containing mixture + *L. plantarum* MCC1; additive cowberry concentrated juice drink (C).
Dietary supplement I (or DS/FP-I): Milk protein containing mixture + *L. plantarum* MCC1 + *L. gasseri* MCC2; additive sea-buckthorn juice (BT);
Dietary supplement II (or DS/FP-II): Milk protein containing mixture + *L. plantarum* MCC1; additive raspberry-blueberry concentrated juice drink (RB).
Dietary supplement III (or DS/FP-III): Milk protein containing mixture + *L. gasseri* MCC2; additive.

**Table 10.**

| Example of typical organoleptical test of food product. Degustators (n = 15). The next parameters were asked to be evaluated: consistency, colour, smell, taste and acidity on the basis of the following score: 1 - repulsive, 2 - not too tolerable, 3 - tolerable, 4 - good, 5 - excellent/inviting. | | | | |
|---|---|---|---|---|
| | FP-V | FP-IV | FP-III | FP-I |
| Consistency | 2.3±1.2 | 3.6±1.0 | 3.3±1.3 | 4±1.1 |
| Colour | 2.6±0.9 | 3.8±0.8 | 3±1 | 4.1±1.2 |
| Taste | 2.8±0.9 | 3.2±1.0 | 3.8±0.8 | 3.5±1.0 |
| Smell | 2.6±0.9 | 2.8±1.0 | 3.4±0.8 | 3.4±1.2 |
| Acidity | 2.8±0.9 | 3.1±0.8 | 3.7±0.6 | 3.2±1.2 |
| Total | 2.6±1.0 | 3.3±0.9 | 3.4±0.9 | 3.7±1.2 |

Example 2. Additives for production of milk allergy responses and urinary tract irritation symptoms and associated oxidative stress and inflammation reducing food product and dietary supplement containing microorganisms *L. plantarum* MCC1 or *L. gasseri* MCC2 or their combination.

As product additives sea-buckthorn juice (BT) (made from sea-buckthorn berries, without preservatives, cold-pressed and pasteurized), raspberry-blueberry concentrated juice drink (composition: concentrated raspberry-blueberry juice (RB), sugar, acidity regulator citric acid, preservative potassium sorbate, was diluted 10 times), cowberry concentrated juice drink (C) and blueberry concentrate (B) (see Table 10) were used. Antioxidancy (TAA and DPPH tests) and contents of bioelements with atom absorption spectrophotometric (AAS) method Spektra AAFS and 220Z were determined for the additives. (Varian, Australia), Cu, Zn, Fe, Mn contents were determined with AAS flame method, K content with emission method in air-acetylen mixture. Organoleptical tests of the end-product were carried out according to valid requirements with special questionnaires. The results are presented in Table 10.

### Example 3. Clinical trial no. 1.

Safety and beneficial effects of the products, biochemical-clinical and organoleptic tests.

With the use of multipotent strains obtained by versatile bioselection and additives and application of suitable technological scheme (see example 1) food products FP-I (MCC1+ *L. gasseri* MCC2, BT) and FP-II (MCC1, RB) were obtained. Subsequently clinical trials (in accordance to Declaration of Helsinki of the World Medical Association, approved by the Tartu University Ethics Review Committee) were performed to clarify the safety and healthy effects of food products FP-I and FP-II.

The main scheme of the clinical trial was the following: 25 volunteers (11 women, 14 men), 40-65 years old, were randomly assigned to two groups on the basis of the next study inclusion criteria: persons without clinical problems, chronic diseases, special diets, use of vitamins, mineral preparations, the participants do not have to change their physical activity, usual alcohol use, smoking habits or eating habits. Before and after two-week everyday use of 200 g of FP-I (n=10) or FP-II (n=15) the next biochemical-clinical parameters were determined: inflammation markers (hsCRP, MPO, IL-6, IL-10), lipid and lipoprotein markers (TG, cholesterol, LDL-cholesterol, HDL-cholesterol, sdLDL), immunoglobulins (IgM, IgA, IgG, IgE), oxidative stress (OxS) markers (isoprostanes, oxLDL, LDL-b2GPI), blood glucose, blood pressure and urine creatinine.

### Results

### 1) Safety

Scientific analysis of everyday questionnaire showed that no subjects complained of discomfort or intestinal problems during the two-week use and no negative symptoms occurred. The use of FP-I or FP-II did not have effects that would drive their biochemical and clinical parameters outside the limits endemic reference values. They did not increase the amount of IgE antibodies as one of the important allergy markers compared to the pre-trial period, did not cause inflammatory reaction (hsCRP oxLDL, MPO, IL-6) or change the values of blood lipids, lipoproteins (TG, LDL, HDL) and glucose nor increase the level of OxS in the body (isoprostanes, oxLDL, MPO, sdLDL, LDL b2GPI) (Table 10) and did not damage the renal functions (urine creatinine). Thus: FP-I and FP-II obtained on the basis of the ideology of the invention don't cause inflammatory reactions, general allergic sensibilization, OxS and therefore do not damage the organism in healthy persons.

### 2) Health promoting effects

The analysis of the results of the clinical trial showed that the use of FP-I or FP-II induced some statistically significant and somewhat different positive effects (Table 10). FP-I exerted the effect reducing the systemic OxS load of the whole body (decrease of urine isoprostanes and blood MPO level) and anti-inflammatory effect (decrease of blood MPO). FP-II reduced the systemic OxS load of the whole body (decrease of urine isoprostane level) and exerted anti-inflammatory effect (decrease of hc-CRP and increase of IL-10). At the same time the use of both food products did not change the numerical values of immunoglobulins IgM, IgA and IgM.

Thus: the first clinical trial showed that the ideology of the invention (multivariant purposeful bioselection of the strains, bioselection of additives and technological solution) resulted in food products that are safe and are more hypoallergenic having several inflammation and oxidative stress-related effects (cause less allergic responses compared to cow's milk) and have a positive effect on biochemical and clinical parameters also in persons with prostate complaints (including urination disorders).

**Table 11. Biochemical and clinical parameters (clinical trial no. 1; M± SD)**

| | GROUP I (FP-II)* | | GROUP II (FP-I)** | |
|---|---|---|---|---|
| | Before | After | Before | After |
| Blood pressure, mmHg | 123/74±7/5 | 125/70±9/5 | 118/71±12/5 | |
| Total cholesterol mmol/L | 5.8±1.3 | 5.9± 1.4 | 6.3±1.1 | 6.6±0.9 |
| HDL-cholesterol mmol/L | 1.6±0.4 | 1.6±0.5 | 1.7±0.5 | 1.7± 0.5 |
| LDL-cholesterol | 4.0±1.1 | 4.1±1.2 | 4.6± 1.1 | 4.9±1.1 |
| triglucerides mmol/L | 1.4±1.3 | 1.4± 1.3 | 1.4± 0.5 | 1.5±0.7 |
| Glucose mmol/L | 5.2±0.4 | 5.2±0.7 | 5.2± 0.5 | 5.3±0.5 |
| hs-CRP | 0.72±0.45 | 0.90±0.59 | 3.1±1.8*** | 1.9±1.5*** |
| mg/L | | | | p = 0.049 |
| IgM g/L | 1.26±0.48 | 1.29±0.49 | 1.09±0.37 | 1.10±0.35 |
| IgA g/L | 2.42±0.85 | 2.38±0.87 | 3.06±1.46 | 3.08±1.48 |
| IgE g/L | 52±56 | 53±59 | 88±95 | 77±81 |
| MPO | 67±22 | 51± 13 | 53±14 | 61±16 |
| pmol/L | | p=0.018 | | |
| IL-6 pg/mL | 2.6±1.2 | 2.6±0.9 | 3.9±2.1 | 2.9±0.9 |
| IL-10 pg/mL | 6.8±2.9 | 6.1±2.6 | 6.4± 2.5*** | |
| | | | 7.8±2.9*** | |
| | | | p= 0.04 | |
| oxLDL U/L | 77±33 | 75±33 | 74+24*** | |
| | | | 69± 21*** | |
| | | | p = 0.039 | |
| LDL 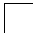 2GPI | 1.4±1.6 | 1.1±1.2 | 1.211.2*** | |
| | | | 1.0+ 0.7*** | |
| sdLDL | 38.6±15.7 | 37.1±16.3 | 39.6±16.1 | 38.8±16.0 |
| 8-isoprostanes ng/mL | 36.7±6.9 | 31.3±12.5 | 49.6± 8.9*** | |
| | | p =0.034 | 37.7± 6.7*** | |
| | | | p=0.021 | |

| | | | | |
|---|---|---|---|---|
| *n = 10, ** n= 9, ***n = 15; blood pressure lowered in four persons in group II | | | | |

### Example 4. Clinical trial no. 2. Cow's milk allergy studies in children

Reactions caused by cow's milk protein occur in 5-15% of infants (Host A. (2002). Frequency of cow's milk allergy in childhood. *Ann Allergy Immunol* 89(Suppl 1):33-7), but cow's milk protein allergy (CMPA) occurs in 2-7.5% (Hill DJ, Firer MA, Shelton MJ et al. (1986) Manifestation of milk allergy in infancy: clinical and immunologic findings. J Pediatr 109: 270-6). CMPA may be IgE- or non-IgE-mediated. As a rule the early reactions are urticaria, angioedema, vomiting or exacerbation of atopic dermatitis. Exacerbation of atopic dermatitis or gastrointestinal complaints occur as late reactions (Vandenplas Y, Brueton M, Dupont C, Hill D, Isolauri E, Koletzko S, Oranje AP, Staiano A. (2007) Guidelines for the diagnosis and management of cow's milk protein allergy in infants. Arch Dis Child. 92:902-908). None of the existing diagnostic tests does prove or deny if the child has CMPA or not (Vanto T, Juntunen-Backman K, Kalimo K et al. (1999) The patch test, skin prick test, and serum milk-specific IgE as diagnostic tools in cows' milk allergy in infants. Allergy 54:837-42). Therefore the diagnostic gold standard of CMPA is elimination diet with subsequent provocation (Vandenplas Y, Brueton M, Dupont C, Hill D, Isolauri E, Koletzko S, Oranje AP, Staiano A. (2007) Guidelines for the diagnosis and management of cow's milk protein allergy in infants. Arch Dis Child. 92:902-908). As a protocol for the provocation test an internationally acknowledged scheme for such investigations described by Isolauri *et al*. was used (Isolauri E, Turjanmaa K. (1996) Combined skin prick and patch testing enhances identification of food allergy in infants with atopic dermatitis. J Allergy Clin Immunol;97:9-15; Sütas Y, Kekki O-M, Isolauri E. (2000) Late onset reactions to oral food challenge are linked to low serum interleukin-10 concentrations in patients with atopic dermatitis and food allergy. Clin Exp Allergy 30:1121-8).

### Methodology

Open provocation with food products FP-II and FP-I was performed in the Centre of Allergic Diseases of the Children's Clinic of the Tartu University Clinics Foundation (in accordance to Declaration of Helsinki of the World Medical Association, approved by the Tartu University Ethics Review Committee). The testing was performed 20 times (part of subjects participated in the trials of both food products) in children aged 9 months to 4 years 8 months (mean age 25.1 months). Pre-trial background was following: all children were diagnosed with cow's milk allergy and/or intolerance by a doctor at the Centre of Allergic Diseases of the Children's Clinic of the Tartu University Clinic Foundation (IgE test to milk, NTT to milk) relying on history and ancillary investigations. All children in the study group were on milk-free elimination diet. After the use of milk 9 children developed skin rash, usually exacerbation of atopic dermatitis, one child developed acute urticaria, one child diarrhoea. One child developed breathing difficulties and skin rash only after eating processed milk products. 3 children had been concurrently diagnosed with asthma, one of them also allergic rhinitis and one pollen allergy (pollinosis). One child had asthma and pollen allergy as concurrent diseases. None of the children received antihistamine or corticosteroid treatment. In three children the concurrent asthma was in remission, therefore they did not receive permanent asthma treatment when the provocation test was performed.

Open oral provocation was performed on the first trial day in the Centre of Allergic Diseases of the Children's Clinic of the TUCF. The child was administered the investigated product (FP-II or FP-I) in increasing amounts (a drop on lower lip, 2 mL, 10 mL, 50 mL, 100 mL). After each amount the child's condition and the development of allergic reaction was evaluated during 20 minutes. If rash, cough, breathing difficulties or abdominal pain/diarrhoea developed, the administration of the product was immediately discontinued. If the child did not have reaction on the first trial day after up to 100 ml of product, the child then received the investigated product at home 100 mL/day on the second, third and fourth day. The parent was asked to evaluate the development of late-onset reactions. The test was considered positive if the child had: a) early reaction - on the first provocation day after administration of small amount of product pruritus, rash, hives or vomiting developed; b) late-onset reaction - dermatitis exacerbation, vomiting, abdominal pain, and diarrhoea developed > 24 hours after initiating the investigated product provocation. The open provocation test was negative if during the provocation (four days) and during subsequent week no reaction developed. Thus the subject tolerated the product.

### Summary of results

The solving of the problem of food allergenicity is very complicated and it is not possible to create products that are absolutely allergic reaction-free but with very high bioquality. The problem becomes more difficult as currently a child can have an allergic reaction to several food products (but also to other common factors) at the same time. This was also observed in our trials (see Table 5), which makes it difficult to exclude that at the given moment the positive response rash did not manifest due to initiation by other causes. Therefore the focus of the investigation was directed so that less allergic responses would result, i.e. the obtained food products would be more hypoallergenic than cow's milk. Each percent of children who are allergy-free or less allergic to given food products would increase the number of children who would get sufficient biologically high-quality food for their development during their most important period of biological development. The provocation test showed that both food product FP-II as well as FP-I were more hypoallergenic than cow's milk (Table 5). At least 3-4 children from 12 did not develop any allergic reaction after FP-I use on the basis of the provocation test. Three subjects out of eight did not develop any allergic reaction after FP-II use. Out of five subjects in whom FP-I caused some reaction, three tolerated FP-II (60%). FP-II was thus in terms of cases even more hypoallergenic than FP-I. Most of the children also preferred FP-II due to taste properties.

**Table 12. Investigation of allergic response to cow's milk (hypoallergenicity trial)**

| B/ G | Diagnosis Clinical manifestation | NTT to milk | NTT to FP-I | Provocation test to FP-I | Other NTT | Age |
|---|---|---|---|---|---|---|
| G1 | CMA, AD skin rash | neg | neg | neg | egg yolk egg white | 23m |
| B2 | CMA, BA, AD (30m) skin rash, breathing difficulty | pos | neg | neg | - | 36m |
| B3 | CMA, AD, BA (24m), pollinosis, skin rash | pos | pos | positive, (from 50 mL) rash on cheeks | egg, nuts, cat, pollen, house dust mite etc | 46m |
| B4 | CMA, AD skin rash | pos | pos | pos (from 10 mL, rash) | egg yolk egg white | 16m |
| B5 | CMA, AD, BA (27m) diarrhoea | neg | neg | pos (on the second day, rash) | egg white | 28m |
| G6 | CMA, AD skin rash | neg | neg | pos (on the third day, rash) | egg yolk egg white | 9m |
| G7 | AD skin rash | neg | neg | pos (on the second day, rash, abdominal pain) | Neg | 18m |
| B8 | CMA, AD skin rash | pos | neg | neg | Neg | 40m |
| B9 | CMA, AD skin rash | pos | pos | pos (from 10 mL, rash) | egg yolk, egg white | 14m |
| B10 | CMA, AD skin rash | pos | neg | neg (on the first day, on the second didn't want to drink) | Neg | 31m |
| B11 | Urticaria hives | pos | neg | neg/pos (days 2-4 diarrhoea) | egg yolk, egg white | 14m |
| B12 | CMA, AD pruritus | pos | pos | pos (on the second day pruritus) | egg yolk egg white | 33m |
| | | | | | | |
| B/G | Diagnosis Clinical manifestation | NTT to milk | NTT to FP-II | Provocation test to FP-II | Other NTT | Age |
| B9 | CMA, AD skin rash | neg | pos | neg | egg yolk, egg white | 23m |
| B3 | CMA, AD, BA (24m), pollinosis, skin rash | pos | pos | pos (on the second day pruritus, days 3 and 4 overexcitability ) | egg, nuts, cat, pollen, house dust mite etc | 56m |
| B11 | Urticaria hives | pos | neg | neg (on the first day diarrhoea after 100 mL, days 2-4 without problems) | egg yolk, egg white | 16m |
| B12 | CMA, AD pruritus | pos | pos | pos (on the first day itching rash) | egg yolk egg white | 34m |
| G7 | AD skin rash | neg | neg | neg | neg | 27m |
| B13 | CMA, AD skin rash | pos | pos | neg | egg yolk egg white | 12m |
| B14 | CMA, AD skin rash | pos | neg | pos (on the second day, rash) | egg yolk, egg white | 18m |
| B15 | AD skin rash | pos | neg | neg | egg yolk, egg white | 17m |

| | | | | | | |
|---|---|---|---|---|---|---|
| CMA - cow's milk allergy, AD - atopic dermatitis, BA - bronchial asthma | | | | | | |

### Example 5. Clinical trial no. 3. The effect of food products FP-I and FP-II on the lower urinary tract irritation symptoms accompanying benign prostatic hyperplasia and on oxidative stress and inflammation indicators.

Abovementioned clinical trials showed that the ideology of the invention (multivariant purposeful bioselection of the strains, bioselection of additives and technological solution) resulted in food products FP-I and FP-II that are safe for use and are more hypoallergenic having several inflammation and oxidative stress-related effects and have a positive effect on urinary tract irritation symptoms and OxS and inflammation indicators.

### Methodology

In the trial participated 55 men (administered FP-I) and 5 men (administered FP-II) who had mild or moderate IPSS (*International Prostate Symptom Score*) < 8. The IPSS questionnaire contains 3 questions assessing the lower urinary tract irritation symptoms and 4 questions assessing urinary obstruction (obstruction symptoms). Also required prior tests (PSA, prostate secretion) were carried out for accurate selection of the study group. It means that with the help of prostate secretion investigations and NIH-CPSI questionnaire active prostatitis (prostate inflammation) was ruled out and by using PSA test and digital palpation the clinical possibility of prostate cancer was ruled out. Thus we included in the study group men with mild or medium urinary disorders in whom other causes of complaints (prostatitis, prostate cancer) were ruled out. The patients (with mild and moderate urination disorder, without inflammation reaction) used food product FP-I or FP-II 200 g daily for two weeks.

### Results

FP-I. Two test series were carried out. In the first 23 patients participated and in the second (where placebo control was also performed) 32 patients (Table 13). On both occasions statistically significant reduction of inflammation marker HsCRP and OxS marker isoprostanes was observed. The second test series also pointed out statistically positive changes of anti-inflammatory IL-10 and inflammation/OxS marker oxLDL. In the first test series (n=23) also the trend of positive changes of this marker was observed. In the second test series also the level of glycated hemoglobin was determined, the decrease of which was statistical.

When summarizing the results of both test series (Table 13), then hsCRP, isoprostanes and oxLDL decreased statistically and there was a positive trend of IgE decrease and IL-10 increased statistically. There was a correlation between hsCRP and oxLDL after the use of FP-I. In control group the statistically significant changes were not seen for any of the mentioned parameters.

FP-II. One test series was performed and the next parameters determined: IgE kU/L, oxLDL, IL-10, 8-isoprostanes, hsCRP and MPO. It was found that in the group of RP-II users statistically significant reduction of inflammation and OxS marker MPO (before 77±13 and after 53±9 ng/mL, p=0.0056) and the reduction of the frequency of urinary disorders in more than 40% of users occurred.

### Summary of results

The administration of FP-I as well as FP-II reduced the level of OxS and inflammation in subjects who had moderate lower urinary tract complaints (IPSS score <8) and had leading irritation symptoms. In case of FP-I in 66% of study group men (55), who had moderate lower urinary tract complaints (IPSS score <8) and leading irritation symptoms, the reduction of complaints for at least 20% occurred and in 30% of study group reduction of complaints for an average of 40% occurred. The clinical and biochemical parameters of the patients (hs-CRP, IL-10, oxLD, 8-isoprostanes) improved statistically possibly. No changes occurred in the control group.

**Table 13. Test series of FP-I (M±SD)**

| | First test series (n=23) | Second test series (n=32) | Total (n=55) |
|---|---|---|---|
| | Before | Before | Before |
| | After | After | After |
| hsCRP mg/L | 2.09±1.06 | 1.92±1.24 | 1.99±1.17 |
| | 1.04±0.90 | 1.57±1.10 | 1.50±0.96 |
| | p=0.016 | p=0.060 | p=0.002 |
| B-Hb A1c, % total Hb 4.5-5.7 | Not determined | 5.87±0.35 | |
| | | 5.79±0.36 | |
| | | p=0.0013 | |
| IgE, kU/L | 98.3±67.5 | 129.8±133.4 | 116.2±107.0 |
| | 86.6±56.1 | 130.6±129.3 | 112.5±99.8 |
| oxLDL, U/L | 64±12 | 72±16 | 68 ± 15 |
| | 63±13 | 70±16 | 65 ± 15 |
| | | p=0.047 | p=0.045 |
| IL-10 | 5.0±1.7 | 8.6±2.2 | 7.1 ±2.6 |
| | 5.6±2.5 | 10.0±2.8 | 8.2±3.3 |
| | | p=0.014 | p=0.012 |
| 8-iso-prostanes to ng/mmol creatinine | 50.8+10.6 | 47.8±15.9 | 48.2±14.2 |
| | 43.3±0.9 | 37.2±10.1 | 39.8±10.2 |
| | p=0.03 | p= 0.0007 | p=0.005 |

### Example 6. The effect of dietary supplement (DS/FP-I) on the lower urinary tract irritation symptoms accompanying benign prostatic hyperplasia and on oxidative stress and inflammation indicators.

10 men who had mild or moderate urination disorder - IPSS (International Prostate Symptom Score) were included in the trial, whereas on the basis of prior prostate secretion investigations and NIH-CPSI questionnaire active prostatitis was excluded and by using PSA test and digital palpation the possibility of clinically significant prostate cancer was excluded. The dietary supplement was used 45 g a day for 14 days. In all 10 patients with mild (IPSS score 2-7) and moderate (IPSS score 8-19) urinary disorders, 3/7 respectively, the urinary disorders improved after the use of dietary supplement (p=0.003) according to the "International Prostate Symptom Score" by 50-66%, whereas it was found that significant positive change occurred in reduction of the need to urinate during the night (average 87.5%).

### Overall summary of all clinical trials

Food products and dietary supplements containing *L. plantarum* MCC1 and *L. gasseri* MCC2 are hypoallergenic, reduce the level of OxS and have statistically valid effect on prostate complaints in those who have moderate lower urinary tract complaints and leading irritation symptoms.

## Claims

1. Isolated microorganism strain *L. gasseri* MCC2 DSM 23882.

2. The microorganism according to claim 1 in killed form.

3. The use of microorganism according to claims 1 and/or 2 as antioxidant proteolytic ingredient for the production of food product and/or dietary supplement.

4. The use of microorganism according to claims 1-2 for the production of hypoallergenic, milk allergy reducing food product and/or dietary supplement.

5. The use of microorganism according to claims 1-2 for the production of food product and/or dietary supplement reducing lower urinary tract irritation symptoms accompanying benign prostatic hyperplasia and oxidative stress and inflammation associated with these.

6. A composition comprising microorganism according to claims 1 and/or 2.

## Patentansprüche

1. Isolierter Mikroorganismenstamm *L.Gasseri* MCC2 DSM 23882.

2. Mikroorganismus nach Anspruch 1 in getöteter Form.

3. Verwendung des Mikroorganismus nach einem der vorangehenden Ansprüche 1 und/oder 2 als antioxidativer und proteolytischer Bestandteil zur Herstellung eines Nahrungsproduktes und/oder eines Nahrungsergänzungsmittels.

4. Verwendung des Mikroorganismus nach einem der vorangehenden Ansprüche 1 - 2 zur Herstellung eines hypoallergenen, die Milchallergie vermindernden Nahrungsproduktes und/oder eines Nahrungsergänzungsmittels.

5. Verwendung des Mikroorganismus nach einem der vorangehenden Ansprüche 1 - 2 zur Herstellung eines Nahrungsproduktes und/oder eines Nahrungsergänzungsmittels, das die Symptome der Reizung am den unteren Harnwegen vermindert, welche mit der benignen Prostatahyperplasie und dem oxidativen Stress und der damit verbundenen Entzündung einhergehen.

6. Zusammensetzung, umfassend einen Mikroorganismus nach einem der vorangehenden Ansprüche 1 und/oder 2.

## Revendications

1. La souche isolée de microorganisme *L. gasseri* MCC2 DSM 23882.

2. Le microorganisme selon la revendication 1 sous la forme tuée.

3. L'utilisation du microorganismes selon la revendication 1 et / ou 2 en tant que l'ingrédient antioxydant protéolytique destiné pour la production d'un produit alimentaire et / ou d'un complément alimentaire.

4. L'utilisation du microorganisme selon les revendications 1 - 2 pour la production des hypoallergéniques, d'un produit alimentaire réduisant l'allergie au lait et / ou d'un complément alimentaire.

5. L'utilisation de microorganisme selon les revendications 1 - 2 pour la production d'un produit alimentaire et / ou d'un complément alimentaire, réduisant les symptomes d'irritation des voies urinaires basses qui accompagnent hyperplasie bénigne de la prostate et le stress oxydatif ainsi queles inflammations associées à ces derniers

6. La composition comprenant le microorganisme selon les revendications 1 et / ou 2.
